# EUROPEAN PATENT APPLICATION

(11) **EP 2 025 333 A1**
(43) Date of publication of application: **18.02.2009**
(21) Application number: 07114341.6
(22) Date of filing: 14.08.2007
(51) Int. Cl.: A61K 9/107, A61K 9/00, A61K 47/10, A61K 47/24, A61K 47/44

(54) **Injectable pharmaceutical formulation of taxoids**

(71) Applicant: Pharmatex Italia Srl, 20121 Milano (IT)
(72) Inventor: De Tommaso, Vincenzo, Dr., 20052 Monza (IT)
(74) Representative: Serravalle, Marco

(57) **Abstract**

The present invention relates to injectable pharmaceutical formulations of Taxoids, for example Paclitaxel or Docetaxel, wherein the taxoid is present in an oil-in-water emulsion comprising an edible oil, a phospholipid and a viscosity improver, and wherein the weight ratio of oil to taxoid is lower to or equal than 50:1. The invention also relates to the process for the preparation of these emulsions.

## Description

The present invention relates to injectable pharmaceutical formulations of Taxoids, for example Paclitaxel or Docetaxel.

Taxoids are antitumor agents which are usually administered by injection, but due to their poor solubility in water it is difficult to prepare formulations which are acceptable from a therapeutic stand point.

At the present, commercially available formulations of Paclitaxel comprise a surfactant (Cremophor EL^{™} or Tween 80^{™}) and ethanol. Surfactants are very dangerous for the human body, since they produce side effects such as hypersensitivity. Furthermore, they produce the extraction of DEHP from plastic containers containing PVC. It is therefore necessary to carefully avoid the use of any plastic which contains PVC.

In the recent years many attempts have been made to prepare different formulations which do not contain surfactants. US 2006/067952 discloses an injectable oil-in-water emulsion comprising a taxoid drug, an oil component, a phospholipid component and water. Although the application defines the emulsion as "low-oil", the ratio between oil and taxol is, in the examples, higher than 60:1. It means that for each mg of taxol, the emulsion contains more than 60 mg of oil.

The present invention relates to new formulations of taxoids, wherein the taxoid is present in an oil-in-water emulsion, characterized by the fact that the ratio oil:taxoid is lower than 50:1, preferably lower than 40:1. More specifically, the emulsions of the present invention comprise a taxoid, water, oil, a phospholipid and a viscosity improver.

Furthermore, the emulsions according to the present invention reduce the overall amount of organic compounds present in the emulsion itself. In a preferred embodiment, the weight ratio of Taxoid versus the sum of all organic compounds present in the emulsion is lower than 80:1, preferably lower than 60: 1.

The present invention allows the preparation of oil-in-water injectable emulsions with a concentration equal to or higher than 6 mg/ml, stable in time and easy to dilute, as it is usually done in therapy, to a final concentration comprised between 0.3 mg/ml and 1.2 mg/ml.

In the present invention it is possible to use any edible oil commercially available. Examples of edible oils are rapeseed oil, safflower oil, olive oil, soybean oil, corn oil, cottonseed oil, almond oil, peanut oil, sesame oil, coconut oil, palm oil, etc.. The preferred oil is soybean oil. The amount of oil used in the preparation of the emulsion is typically higher than 5% by weight, preferably higher than 8% by weight, most preferably higher than 10% by weight, even more preferably higher than 15% by weight.

With the term "phospholipid" it is intended a triester of glycerol with two fatty acids and one phosphate ion. Exemplary phospholipids useful in the present invention are phosphatidyl choline, lecithin (e.g. soybean lecithin and egg lecithin), phosphatidylethanolamine, phosphatidylglycerol. Any type of lecithin can be used in the preparation of the oil-in-water emulsion of the present invention. A preferred phospholipid for use in the present invention is soybean lecithin. The amount of phospholipid can vary in a broad range, it is however preferably comprised between 0.5 and 10 % by weight, most preferably between 1 and 5% by weight.

A viscosity improver is used in the preparation of the formulation. With viscosity improver it is intended a compound which is able to increase viscosity in the emulsion. In this way, the emulsion is more stable. Amongst the possible viscosity improver we can cite glycerol, dimethyl sulfoxide (DMSO), propylene glycol and glycofurol. Glycerol is the preferred viscosity improver. The amount of viscosity improver can vary in a broad range, and preferably its concentration in the emulsion can vary from 1 to 25% by weight, preferably from 1.5 to 15% by weight, most preferably from 2 to 10 % by weight.

The formulation according to the present invention is preferably prepared according to the following process:
a) Solubilization of the phospholipid and taxoid in oil, optionally with the aid of ethanol;
b) Separately, solubilization of the viscosity improver in water;
c) The solution of a) is sterilized by filtration with a PTFE hydrophobic membrane with a size of 0.22 micron, in sterile room (Class A); if ethanol is present, it is stripped under vacuum;
d) In another vessel, the water solution of b) is sterilized by filtration with a PTFE hydrophilic membrane with a size of 0.22 micron, in sterile room (Class A).
e) The sterile solution of d) is added to the sterile solution of c) at a temperature of 40-50 °C under vigorous stirring;
f) The obtained emulsion is homogenized under pressure or by the sonication until the size of the oily droplets is lower than 1 micron;
g) The taxoid emulsion is bottled sterilely.

In another preferred embodiment the oil emulsion is obtained according to the following process:
a) Solubilization of the phospholipid in oil;
b) The solution of a) is sterilized by filtration with a PTFE hydrophobic membrane with a size of 0.22 micron, in sterile room (Class A);
c) To the sterile solution of b) a sterile taxoid is added;
d) Solubilization of the viscosity improver in water;
e) In another vessel, the water solution of d) is sterilized by filtration with a PTFE hydrophilic membrane with a size of 0.22 micron, in sterile room (Class A).
f) The sterile solution of e) is added to the sterile solution of c) at a temperature of 40-50 °C under vigorous stirring;
g) The obtained emulsion is homogenized under pressure or by sonication until the size of the oily droplets is lower than 1 micron;
h) The taxoid emulsion is sterilely filled into glass vials.

The formulation according to the present invention presents several advantages in view of the formulations of the prior art:
1) Absence of dangerous ingredients, e.g. surfactants.
2) Easiness of use for paramedicals.
3) Absence of interaction of the emulsion with the plastic used for the intravenous administration.
4) Easiness of the production process, which does not require a specific apparatus, but it follows standard process procedures for sterile products.

### Examples

### Example 1

600 mg of Paclitaxel and 2.4 g of Soybean lecithin (Epikuron 200 ^{™}) were dissolved in 20 g of Soybean oil (purified) and 10 ml of absolute ethanol at a temperature comprised between 40 and 50 °C, under mild stirring. The solution was sterilized by filtration with a PTFE hydrophobic membrane with a size of 0.22 micron, in sterile room (Class A). Ethanol was removed under vacuum at 40-50°C. In another vessel, 4.5 g of glycerol were dissolved in 65 ml of water for injectable solutions. The solution was sterilized by filtration with a PTFE hydrophilic membrane with a size of 0.22 micron, in sterile room (Class A). The solution of Paclitaxel was placed under vigorous stirring and the sterile water-glycerol solution was added. The emulsion was left under vigorous stirring at a temperature comprised between 25 and 35 °C, giving rise to an emulsion. The emulsion was homogenized by using a high pressure homogeniser (850-900 bar) or by sonication. The homogenisation is repeated until all oil droplets have a size lower than 1 micron. Sterile water is added in order to bring the final volume to 100 ml. The concentration of Paclitaxel was 6 mg/ml (which corresponds to the concentration of the commercially available Paclitaxel). The pH of the emulsion was checked to verify that it was comprised between 4.0 and 7.5. The emulsion was kept under stirring for 20' and was then sterilely filled in glass vials of 5 ml, 16.7 ml, 25 ml and 50 ml.

### Example 2

2.4 g of Soybean lecithin (Epikuron 200 ^{™}) were dissolved in 20 g of Soybean oil (purified) at a temperature comprised between 85 and 90 °C, under mild stirring. The solution was sterilized by filtration with a PTFE hydrophobic membrane with a size of 0.22 micron at a temperature of 60 °C, in sterile room (Class A). In accordance with the European Pharmacopeia GMP Annex 1, update of September 2003, 600 mg of sterile powder of Paclitaxel were introduced in a sterile room (Class A) and solubilized at a temperature comprised between 90 and 100 °C in the oily phase. In another vessel, 4.5 g of glycerol were dissolved in 65 ml of water for injectable solutions. The solution was sterilized by filtration with a PTFE hydrophilic membrane with a size of 0.22 micron, in sterile room (Class A). The solution of Paclitaxel was placed under vigorous stirring at a temperature of 95-100°C and the sterile water-glycerol solution was added. The emulsion was left under vigorous stirring at a temperature comprised between 25 and 35 °C, giving rise to an emulsion. The emulsion was homogenized by using either a high pressure homogeniser (850-900 bar) or sonication. The homogenisation is repeated until all oil droplets have a size lower than 1 micron. Sterile water is added in order to bring the final volume to 100 ml. The concentration of Paclitaxel was 6 mg/ml (which corresponds to the concentration of the commercially available Paclitaxel). The pH of the emulsion was checked to verify that it was comprised between 4.0 and 7.5. The emulsion was kept under stirring for 20' and was then sterilely filled into glass vials of 5 ml, 16.7 ml, 25 ml and 50 ml.

### Comparative Example 3

A Paclitaxel solution similar to the commercial product was prepared by dissolving Paclitaxel in a 50/50 mixture of Cremophor EL ^{™} and ethanol. The concentration of Paclitaxel was 12.5 mg/ml.

### Example 4 In vivo effect of the formulation of the invention.

The formulation of example 2 was used in the treatment of mice and compared with the activity of the formulation of comparative example 3. The formulations were diluted immediately before use to a concentration of Paclitaxel of 2.5 mg/ml by using physiologic solution.

56 female nude mice purchased from Harlan Italy approximately 7 weeks old were injected with 10x10⁶ human ovaric tumor cells A2780. When the tumoral masses reached the weight of 150-200 mg, the mice were randomly divided into four groups treated in the following way: a) blank of example 2, b) example 2, 25 mg/kg, c) blank of comparative example 3, d) comparative example 3, 25 mg/kg.

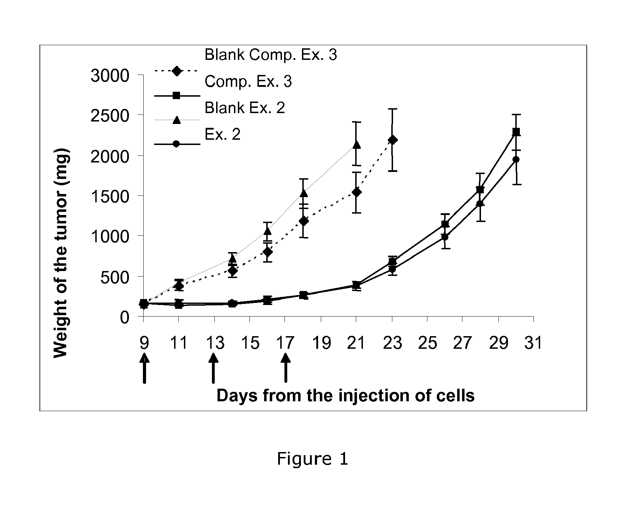

All mice were treated 3 times after 9, 13 and 17 days from the date of injection of the cells. Tumoral growth was evaluated by measuring the size (length D and width d) with a calibre. The volume V of the tumor was calculated using the formula V = (d²xD)/2 and the weight was calculated assuming the density of the tumor equal to 1 g/cm³. Mice were sacrificed when reaching a tumoral mass of about 2 g. Figure 1 reports the results.

Each point in the graph is the average value from 10 mice. The arrows indicate the day in which the treatment took place.

## Claims

1. Pharmaceutical injectable formulation of taxoids wherein the taxoid is present in an oil-in-water emulsion comprising an edible oil, a phospholipid and a viscosity improver, and wherein the weight ratio of oil to taxoid is lower to or equal than 50: 1.

2. Formulation according to claim 1 wherein the taxoid is selected from Paclitaxel and Docetaxel.

3. Formulation according to claims 1-2 wherein the viscosity improver is glycerol.

4. Formulation according to any of the preceding claims wherein the weight ratio of oil to taxoid is lower to or equal than 40:1.

5. Formulation according to claims 1-4 wherein the phospholipid is selected from phosphatidyl choline, egg lecithin and soybean lecithin.

6. Formulation according to claims 1-5 wherein the amount of phospholipid is comprised between 0.5 and 10% by weight, preferably between 1 and 5% by weight.

7. Formulation according to claims 1-6 wherein the weight ratio of Taxoid versus the sum of all organic compounds present in the emulsion is lower than 80: 1, preferably lower than 60: 1.

8. Formulation according to claims 1-7 wherein the amount of viscosity improver is comprised between 1 and 25% by weight.

9. Process for the preparation of the pharmaceutical injectable formulations of claims 1-8 according to the following process:
a) Solubilization of the phospholipid and taxoid in oil, optionally with the aid of ethanol;
b) Separately, solubilization of the viscosity improver in water;
c) The solution of a) is sterilized by filtration with a PTFE hydrophobic membrane with a size of 0.22 micron, in sterile room (Class A); if ethanol is present, it is stripped under vacuum;
d) In another vessel, the water solution of b) is sterilized by filtration with a PTFE hydrophilic membrane with a size of 0.22 micron, in sterile room (Class A).
e) The sterile solution of d) is added to the sterile solution of c) at a temperature of 40-50 °C under vigorous stirring;
f) The obtained emulsion is homogenized under pressure or by the sonication until the size of the oily droplets is lower than 1 micron;
g) The taxoid emulsion is sterilely filled into glass vials

10. Process for the preparation of the pharmaceutical injectable formulations of claims 1-8 according to the following process:
a) Solubilization of the phospholipid in oil;
b) The solution of a) is sterilized by filtration with a PTFE hydrophobic membrane with a size of 0.22 micron, in sterile room (Class A);
c) To the sterile solution of b) a sterile taxoid is added;
d) Solubilization of the viscosity improver in water;
e) In another vessel, the water solution of d) is sterilized by filtration with a PTFE hydrophilic membrane with a size of 0.22 micron, in sterile room (Class A).
f) The sterile solution of e) is added to the sterile solution of c) at a temperature of 40-50 °C under vigorous stirring;
g) The obtained emulsion is homogenized under pressure until the size of the oily droplets is lower than 1 micron;
h) The taxoid emulsion is sterilely filled into glass vials.
